(19) Europäisches Patentamt
European Patent Office
Office européen des brevets

(11) Publication number: **0 196 732 B1**

(12) **EUROPEAN PATENT SPECIFICATION**

(45) Date of publication of the patent specification: **01.03.89**

(51) Int. Cl.[4]: **B 01 J 23/89**, B 01 J 27/24, C 07 C 29/15, C 07 C 1/04, C 10 G 69/02

(21) Application number: **86200700.2**

(22) Date of filing: **17.12.82**

(60) Publication number of the earlier application in accordance with Art. 76 EPC: **0082692**

(54) **Process for upgrading synthesis gas and catalysts therefor.**

(30) Priority: **21.12.81 US 332771**
**21.12.81 US 332772**

(43) Date of publication of application: **08.10.86 Bulletin 86/41**

(45) Publication of the grant of the patent: **01.03.89 Bulletin 89/9**

(84) Designated Contracting States: **BE DE FR GB IT NL**

(56) References cited:
**FR-A- 2 231 416**
**GB-A- 1 219 281**
**GB-A- 2 074 164**
**US-A- 3 901 827**
**US-A- 3 953 363**
**US-A- 4 085 157**
**US-A- 4 235 798**

(73) Proprietor: **THE STANDARD OIL COMPANY, 200 Public Square, 36-F-3454, Cleveland Ohio 44114-2375 (US)**

(72) Inventor: **Pesa, Frederick Anthony, 764 Circlewood Drive, Aurora Ohio 44202 (US)**
Inventor: **Graham, Anne Marie, 6290 Greenwood Parkway, 103 Northfield Ohio 44067 (US)**

(74) Representative: **Smith, Sydney et al, Elkington and Fife Beacon House 113 Kingsway, London WC2B 6PP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 196 732 B1

## Description

The present invention is directed to the upgrading of synthesis gas tp produce mixtures of hydrocarbons and to catalysts of use in such process.

In particular, the present invention is directed to a vapour phase reaction of synthesis gas comprising carbon monoxide and hydrogen in the presence of a catalyst to produce mixtures of hydrocarbon and oxygenated hydrocarbons. It also relates to catalysts of use in such process.

U.S.-A-2 476 788 discloses the synthesis of hydrocarbons, including oxygenates such as aldehydes, ketones and alcohols from carbon monoxide and hydrogen in the presence of metals or metal oxides selected from nickel, iron or cobalt, optionally with promoter metals or metal compounds of aluminium, cerium, magnesium, manganese, thorium, titanium, uranium, zinc, and zirconium. The catalyst could be supported on suitable carriers such as clay, silica gel, and alumina.

U.S.-A-2 535 060 and USA-2 549 470 disclose the preparation of straight-chain primary hydroxyalkanes by introducing hydrogen, carbon monoxide and a hydroxylated solvent into a reaction vessel and heating the mixture in the presence of a ruthenium-containing catalyst (particularly ruthenium metal, oxide, carbonyl, or salts of carboxylic acids which give rise to formation of the carbonyl) and in US-A-2 549 470 in the presence of an alkaline reagent by maintaining pH in the range of 7.0 to 11.5. Both Patents teach that it is essential that the reaction take place in the liquid phase.

U.S.-A-3 941 819 describes the production of ethane, ethylene and dimethyl ether by passing a carbon monoxide and hydrogen mixture over platinum supported on alumina.

U.S.-A-4 014 913 discloses the preparation of acetic acid, ethanol and acetaldehyde by contacting $H_2$ and CO with a rhodium-manganese catalyst.

U.S.-A-4 080 262 describes the production of hydrocarbon mixtures by contacting a mixture of carbon monoxide and hydrogen with a carbon monoxide reduction catalyst and an acidic crystalline alumino silicate (zeolite). Chang-et al. teach that prominent types of catalysts include metals or axides of Zn, Fe, Co, Ni, Ru, Th, Rh, and Os, and that «with the exception of ruthenium, all practical art recognized synthesis catalysts contain chemical and structural promotors».

U.S.-A-4 096 164 discloses the production of oxygenated 2 carbon atom hydrocarbons by reacting CO and $H_2$ in the presence of catalysts comprising Rh, Mo and W.

U.S.-A-4 101 460 and U.S.-A-4 136 104 disclose the conversion of synthesis gas to acetic acid and relates 2 carbon atom oxygenated derivatives in the presence of a rhodium metal/ruthenium metal catalyst.

U.S.-A-4 116 994 discloses the selective production of olefinic hydrocarbons from carbon monoxide and hydrogen using a catalyst comprising rhodium deposited on titanium containing oxides.

U.S.-A-4 119 656 describes the production of one to 2 carbon atom oxygenated hydrocarbons by contacting synthesis gas with a catalyst consisting essentially of palladium.

U.S.-A-4 122 110 discloses the manufacture of linear saturated primary alcohols from synthesis gas using a catalyst comprising copper, cobalt, a third metal selected from chromium, iron, vanadium and manganese, at least one alkali metal and optionally zinc.

U.S.-A-4 162 262 discloses the production of 2 carbon atom oxygenated hydrocarbons while minimizing co-production of methanol by reacting $H_2$ and CO with a catalyst containing rhodium metal, uranium or thorium and optionally iron, molybdenum or tungsten.

U.S.-A-4 171 320 discloses the selective production of olefins from carbon monoxide and hydrogen using as a catalyst ruthenium on a support comprising at leat one refractory Group VB metal oxide.

U.S.-A-4 199 522 discloses the preparation of olefins of 2 to 4 carbon atoms from carbon monoxide and hydrogen using catalysts comprising a sulfide, oxide or metal of Mo, W, Re, Ru, Ni, Pd, Rh, Os, Ir or Pt and a hydroxide, oxide or salt of Li, Na, K, Rb, Cs, Mg, Ca, Sr, Ba or Th.

U.S.-A-4 201 597 discloses the preparation of oxygenated hydrocarbons by reacting carbon monoxide and hydrogen in the presence of a catalyst containing rhodium, tungsten and an alkali metal.

U.S.-A-4 206 134 discloses the selective preparation of low weight olefins from carbon monoxide and hydrogen using as a catalyst ruthenium on a support consisting of a manganese-containing oxide.

U.S.-A-4 235 801 discloses the preparation of ethanol by contacting a synthesis gas mixture containing CO and $H_2$ with a rhodium-iron catalyst.

U.S.-A-4 246 186 discloses the preparation of two carbon atom oxygenated hydrocarbons from hydrogen and carbon monoxide by reaction with a rhodium metal catalyst, as compared to other single element Group VIII metal and copper catalysts.

EP-A-18 763 describes the production of oxygenated hydrocarbons having 1 to 4 carbon atom by reaction of CO and $H_2$ in the presence of a catalyst comprising rhodium, chromium and optionally Fe, Mn, Mo, W or Ru. The catalyst may be prepared upon a support which has been formerly activated by the addition of metals or non-metals such as alkalis, Th, Mn, Rh, Fe, Cr, Mo, B, and P.

EP-A-4 653 and EP-A-4 656 describe the production of acetic acid, ethanol and acetaldehyde by reacting CO and $H_2$ with a catalyst containing rhodium, magnesium and a halide.

We have found that catalysts comprising the mixed metal oxides of ruthenium, copper, at least one alkali metal, and at least one rhodium, iridium, palladium or platinum are useful for the upgrading of synthesis gas to hydrocarbons, exhibiting good selectivity to alkanes and oxygenated hydrocarbon products, particularly alcohols, with high selectivity.

The invention provides such catalysts and processes for upgrading synthesis gas utilizing such catalysts.

In one embodiment, the process of the present invention includes the upgrading of synthesis gas to obtain selectivity to alkanes and alcohols comprising

contacting carbon monoxide and hydrogen in the vapour phase at a reaction temperature of at least 250°C and a reaction pressure of at least 500 psi (3.44 MPa) with a catalyst of the formula

$$A_aRu_bCu_cM_dN_zO_x$$

wherein
A is an alkali metal,
M is R, Ir, Pd, Pt or mixtures thereof and
wherein
a is about 0.002 to about 0.5,
b is about 0.5 to about 3,
c is about 0.5 to about 3,
d is about 0.05 to about 0.5,
z is a level of 0 to about 1 weight % and
x is the number of oxygens needed to fulfill the valence requirements of the other elements.

In a further embodiment of the invention, the products of the synthesis gas upgrading process are contacted with hydrogen at elevated temperature and pressure in the presence of a hydrogenated catalyst.

The present invention further includes novel catalysts of the composition

$$A_aRu_bCu_cM'_dN_zO_x$$

wherein
A is an alkali metal,
M' is Rh, Ir, Pd, Pt or mixtures thereof, and
wherein
a is about 0.002 to about 0.5
b is about 0.5 to about 3,
c is about 0.5 to about 3,
d is about 0.05 to about 0.5,
z is a level of 0 to about 1 weight % and
x is the number of oxygens needed to fulfill the valence requirements of the other elements.

Thus, in the process of the present invention, synthesis gas, or a mixture of carbon monoxide and hydrogen, is reacted in the presence of a carbon monoxide hydrogenation catalyst in the vapour phase to form hydrocarbons, and in particular, olefins and carboxylic acids in one embodiment of the invention, and alkanes and alcohols in another embodiment of the invention.

Synthesis gas may be produced by means known in the art and practiced commercially, including providing synthesis gas as a product of the partial combustion of coal, natural gas, petroleum bottoms or other carbonaceous materials. One method of derivation is the heating of coke in the presence of air and then steam. The ration of carbon monoxide to hydrogen in the synthesis gas mixture to be upgraded may vary from about 1:10 to 10:1 and is preferably in the range of 1:3 to about 3:1. The synthesis gas may contain a very low amount of sulfur compounds, and may also contain small amounts of carbon dioxide, nitrogen and other inerts.

Although synthesis gas is a preferred reactant, any other gas composed primarily of hydrogen and carbon monoxide and having $CO:H_2$ ratio of 1:10 to 10:1 may be employed. Preferably the gaseous reactant is essentially sulfur free.

*Process Conditions*

The process of the present invention is carried out by contacting the gaseous reactants containing carbon monoxide and hydrogen, with the novel catalyst described below in a suitable fluid bed or fixed bed reactor. The reaction can be conducted continuously or in a batch-type operation. The reaction temperature should be maintained between 250°C to 400°C, preferably 275°C to 375°C.

The reaction pressure should normally be maintained between 500 psi (3.44 MPa) to 5000 psi (34.4 MPa) preferably 500 psi (3.44 MPa) to 1500 psi (10.3 MPa). The reactant gases may be fed to the reactor utilized at a space velocity (litres gaseous reactants fed per litre of catalyst per hour) of 100 per hour to 10,000 per hour, preferably 500 per hour to 6,000 per hour.

The contact time of the reactants with the catalyst is generally between 10 seconds to 200 seconds, and is preferably 15 seconds to 100 seconds.

The novel catalyst provided in one embodiment of the present invention is believed to be am oxide complex and comprises the composition described by the empirical formula

$$A_aRu_bCu_cM'_dN_zO_x$$

wherein
A is an alkali metal
M' is Rh, Ir, Pd, Pt or mixtures thereof, and
wherein
a is 0.002 to 0.5,
b is 0.5 to 3,
c is 0.5 to 3,
d is 0.05 to 0.5,
z is a level of 0 to 1 weight % and
x is the number of oxygens needed to fulfill the valence requirements of the other elements.

M' is preferably rhodium, iridium or palladium, and A may be selected from Na, Li, K, Rb and Cs, although Na, K, and Rb are preferred.

The Ru to Cu ratio is preferably 1:0.75 to 1:1.5. The ratio of ruthenium to the promoter metal (M') is generally 1:0.05 to 1:0.5 and is preferably 1:0.1 to 1:0.5. Alkali metal is required in the catalyst of the present invention. Mixed oxide catalysts of ruthenium and copper which are alkali free produce essentially all methane, at low conversion. The alkali metal may be present in the catalyst at a level of 0.002 to 0.5 moles alkali metal per mole of ruthenium. A level of 0.02 to 0.4 moles alkali metal per mole of ruthenium is preferred.

The catalyst of the present invention is a mixed metal oxide. In the process of the present invention, the catalyst is preferably utilized in a partially reduced state, howerer, the catalyst is not totally reduced to element metal and thus retains its oxide character.

The catalyst may be prepared by conventional means, such as mixing compounds containing the catalyst components in a liquid solution or slurry, such as a water solution or slurry and heating,

recovering the catalyst precursor from the liquid, drying and calcining. Suitable catalyst component containing compounds may include but are not limited to oxides, hydroxides, inorganic salts such as nitrates, phosphates, halides, carbonates, silicates, aluminates, and salts of organic acids such as acetates, formates, butyrates, propionates, benzylates, and the like. Preferred catalysts of the present invention are prepared by recovering the catalyst precursor by adding to the aqueous solution of ruthenium, copper and promoter (if any) components, an alkali metal hydroxide to cause precipitation of the catalyst precursor, heating in the presence of the alkali metal, and thereafter filtering the precipitate.

The catalyst may be formed in a conventional manner, such as tableting, pelleting, or supporting the active catalyst material on a carrier. The carrier is preferably inert, and may include silica, alumina, Alundum, clay, alumina-silica, silicon carbide and the like. The active catalytic material may be coated on the carrier by the method described in U.S. Patent No. 4 077 912 or may be impregnated on the carrier such as by depositing a solution of the catalyst component containing compounds onto a carrier, drying and calcining. Catalyst components may be added to the carrier separately, if desired.

*Products*

Products of the synthesis gas upgrading process include, among others, methane, ethane, propane, butane, ethylene, propylene, butylene, methanol, ethanol, propanol, butanol, pentanol, acetic acid, propanoic acid, butyric acid, valeric acid, and low amount of aldehydes and esters including acetaldehyde and methyl butyrate. These products are useful as chemical feedstocks, or as fuels, such as in gasoline mixtures. Where conversion is maintained at a moderate or low level, these products can be recovered from the reactor effluent, and the remaining synthesis gas recycled to the reaction.

Products of the upgrading of synthesis gas process of the present invention include, among others, propane, ethylene, methanol, ethanol, propanol, butanol, pentanol, acetic acid, propanoic acid, butyric acid, valeric acid. Small amounts of other alkanes, olefins and aldehydes are present in the products of the inventive process in some embodiments. These products are useful as chemical feedstocks, or as fuels, such as in gasoline mixtures. Where conversion is maintained at a moderate or low level, these products can be recovered from the reactor effluent, and the remaining synthesis gas recycled to the reaction.

Alkanes, esters and alcohols are most suitable for use as fuels, such as in gasoline mixtures. Therefore, in one embodiment of the invention, the liquid product mixture obtained from the synthesis gas upgrading process (containing in addition to alcohols and esters, the non-fuel components such as olefins, aldehydes and carboxylic acids) is contacted with hydrogen at elevated temperature and pressure in the presence of a hydrogenation catalyst. The resulting hydrogenation products, alkanes, alcohols and esters are suitable for use as fuel components.

The hydrogenation process may be conducted in the vapour phase, at a reaction temperature of about 150°C to about 450°C and a reaction pressure of about 250 psig (1.72 MPa) to about 5000 psig (34.4 MPa). Any suitable hydrogenation catalyst, such as nickel or copper chromite may be used, although catalysts represented by the formula

$$G_eRu_fD_gE_hO_x$$

wherein

G = Zn, Cd and mixtures thereof;
D = Co, Ni and mixtures thereof;
E = Fe, Cu, Rh, Pd, Os, Pt and mixtures thereof; and

wherein

e = 0 to 1,
f = 0.01 to 3,
g = 0.01 to 3,
h = 0 to 1,
x = the number of oxygens determined by the valence requirements of the other elements

are preferred.

The following Examples (1-9) are given by way of illustration only. In these example the following general procedures were adopted.

*Specific Embodiment - Example 1-10*
*Catalyst Preparation*

In the examples below, catalysts were prepared by the following method. An amount of ruthenium chloride and copper chloride required to give 0.03 moles of each metal and an amount of promoter metal component sufficient to provide the desired mole ratio of promoter metal to ruthenium were dissolved in 250 millilitres of water with stirring for 30 minutes. Aqueous sodium hydroxide (50% by weight) was added dropwise, with stirring, until the pH reached and remained at 8.3 to 8.5 (approximately 7 to 15 millilitres). The resulting slurry was heated near boiling for 30 minutes with constant stirring, then cooled. The pH was adjusted if necessary to 7.5. The mixture was filtered, and washed, and reslurried with subsequent filtering and washing steps until the molar ratio of sodium to ruthenium present was approximately about 0.02 to about 0.2:1. The solid mixed oxide was dried at 125°C for about 16 hours, was calcined for three hours at about 350°C (in air) and was ground to pass 140 mesh (0.105 millimeters).

The catalysts were coated upon alumina-silica supports in the following manner. 25 grams of Norton SA 5223 Alundum, 10/30 mesh (0.595 millimeters-2.00 millimeters) were placed in a vessel. 1.25 grams distilled water was sprayed onto the Alundum which was rolled for approximately 10 minutes and the procedure was repeated. The metal oxide catalysts, in an amount calculated to give a total of 0.015 moles of active metal, were added in two equal portions with 15 minutes rolling after each. The coated catalyst was dried for about 16 hours at 125°C and calcined three hours at 350°C. Catalysts prepared in this manner contain approximately 5 weight percent active metals, 0.01% to 0.1% by weight sodium and have surface areas of about 2 $m^2/g$, with pore volumes of from about 0.06 to about 0.09 cc/g.

The catalysts were partially reduced in the following manner. A stainless steel tube reactor was packed with catalyst, and hydrogen gas was introduced into the reactor at 150-200 cc/min. at atmospheric pressure. The electric block furnace placed around the reactor was increased in 50° increments stepwise until 500°C was reached. The final temperature was maintained for two hours, at which time the reactor was allowed to cool with hydrogen flow being continued.

In one embodiment of the invention, catalysts are nitrided after reduction by contacting the catalyst with ammonia for several hours at atmospheric pressure and a temperature of about 400°C, with subsequent cooling under ammonia. The nitrided catalysts contain up to 1 weight percent nitrogen, as is preferred. Between 0.5 to 1 weight percent nitrogen is most preferred.

The reaction procedure for example 1-9 was carried out as in examples 1-36 of the European Patent Application No. 82 306 754.1 (Publication No. 0 082 692) of which this is a divisional. The results reported in Table V below were calculated as follows;

$$\text{CO Conversion} = \frac{\text{Moles of CO input-moles of CO efflluent} \times 100}{\text{Moles of CO input}}$$

$$\text{Weight \%} = \frac{\text{Weight product identified} \times 100}{\text{Total Product Weight}}$$

Carbon dioxide and water were not considered in the calculations.

The catalysts identified in the example below were prepared according to the catalyst preparation method set forth above. The catalysts were reduced, nitrited and tested for synthesis gas upgrading by the reaction procedure set forth above. Reaction and test results are set forth in the Table below.

*Examples 1-4*

Catalysts of the formula 5% $Na_aRuCuRh_{0.5}N_zO_x$/95% Alundum were prepared and tested according to procedure set forth above. Rhodium chloride was utilized as the promoter element containing compound. Products of the synthesis gas upgrading reaction utilizing these catalysts were predominantly alkanes and alcohols.

*Example 5*

A catalyst of the formula 5% $Na_aRuCuRh_{0.1}N_zO_x$/95% Alundum was prepared and tested according to the procedure of Examples 1 to 4 except that a lower level of rhodium was utilized in the catalyst preparation. The catalyst was selective to alkanes and alcohol production, with olefin and carboxylic acid production increasing.

*Example 6-7*

Catalysts of the formula 5% $Na_aRuCuIr_{0.5}N_zO_x$/95% Alundum were prepared and tested according to the procedure of Examples 1 to 4 except that iridium chloride was substituted for rhodium chloride. Products of the synthesis gas upgrading reaction utilizing these catalysts were predominantly alkanes and alcohols.

*Example 8-9*

Catalysts of the formula 5% $Na_aRuCuPd_{0.5}N_zO_x$/95% Alundum were prepared and tested according to the procedure of Examples 1 to 4 except that palladium acetate was substituted for rhodium chloride in the preparation. Products of the synthesis gas upgrading reaction utilizing these catalysts were predominantly alkanes and alcohols.

*Comparative Example 10*

A catalyst of the formula 5% $Na_aRuCuN_zO_x$/95% Alundum was prepared and tested according to the procedure of Examples 1-4, except that no promoter metal (M') was added in the catalyst preparation. Products of the synthesis gas upgrading reaction utilizing this catalyst were predominantly carboxylic acids.

As an example of the product mixture produced by the inventive process, the products obtained by testing the catalyst of Example 1 are as follows.

| *Products* | *Wt. (g)* |
|---|---|
| Methane | 0.0500 |
| Ethylene | 0.0123 |
| Methanol | 0.1075 |
| Ethanol | 0.0136 |
| Propanol | 0.0027 |
| Butanol | 0.0017 |
| Pentanol | 0.0022 |
| Acetic Acid | 0.0043 |
| Propionic Acid | 0.0055 |
| Butyric Acid | 0.0051 |
| Valeric Acid | 0.0096 |

TABLE

SYNTHESIS GAS UPGRADING USING 5% $M_dNa_aCuRuN_zO_x$/95% ALUNDUM CATALYSTS

| Ex. NO. | $M_d$ Promoter | CO:$H_2$ Ratio | Temp. °C | Pressure Mla [(psig)] | Space Velocity Hr-1 | % CO Conv. | Weight % Useful Products Alkanes | Olefins | Carboxylic Acids | Alcohols |
|---|---|---|---|---|---|---|---|---|---|---|
| 1 | $Rh_{0.5}$ | 7:3 | 360 | 9.06 (1300) | 2000 | 6.2 | 23.3 | 5.7 | 11.4 | 59.5 |
| 2 | $Rh_{0.5}$ | 3:7 | 360 | 9.06 (1300) | 3300 | 2.6 | 86.8 | — | 2.0 | 11.2 |
| 3 | $Rh_{0.5}$ | 3:7 | 350 | 9.06 (1300) | 2000 | 5.5 | 33.6 | — | 10.3 | 56.1 |
| 4 | $Rh_{0.5}$ | 1:1 | 360 | 9.06 (1300) | 2000 | 5.6 | 36.8 | 6.2 | 7.8 | 49.1 |
| 5 | $Rh_{0.1}$ | 3:7 | 350 | 9.06 (1300) | 3300 | 20.5 | 35.2 | 21.3 | 29.1 | 10.6 j |
| 6 | $Ir_{0.5}$ | 3:7 | 360 | 9.06 (1300) | 2000 | 11.6 | 85.4 | — | 1.8 | 12.8 |
| 7 | $Ir_{0.5}$ | 7:3 | 360 | 9.06 (1300) | 2000 | 3.1 | 61.5 | 4.4 | 8.0 | 26.0 |
| 8 | $Pd_{0.5}$ | 3:7 | 350 | 9.06 (1300) | 3300 | 14.0 | 84.6 | — | 0.7 | 14.6 |
| 9 | $Pd_{0.5}$ | 3:7 | 320 | 6.88 (1000) | 5500 | 3.6 | 79.5 | — | 0.3 | 20.2 |
| C10 | — | 3:7 | 320 | 6.88 (1000) | 5500 | 4.0 | 14.3 | 8.3 | 63.0 | 5.2 k |

a = 0.002 - 0.02
j = less than 4% aldehydes and esters (combined) also present
k = 5.5% aldehydes, 3,8% esters
z = 0.5-1 weight % active catalyst.

**Claims**

1. A catalyst of the composition

$$A_aRu_bCu_cM'_dN_zO_x$$

wherein
A is an alkali metal,

wherein
M' is Rh, Ir, Pd, Pt or mixtures thereof and

wherein
a is 0.02 to 5,
b is 0.5 to 3,
c is 0.5 to 3,
d is 0.05 to 0.5,
z is a level of 0 to 1 weight % and
x is the number of oxygens needed to fulfill the valence requirements of the other elements.

2. A catalyst as claimed in claim 1 characterised in that A is selected from sodium, potassium and rubidium.

3. A catalyst as claimed in claim 1 or claim 2 characterised in that a is 0.02 to 0.4.

4. A catalyst as claimed in any of claims 1 to 3 characterised in that b and c are each 1.

5. A catalyst as claimed in any of claims 1 to 4 characterised in that d is 0.1 to 0.5.

6. A catalyst as claimed in any of claims 1 to 5 chacterised in that the catalyst is partially reduced.

7. A catalyst as claimed in any of claims 1 to 6 characterised in that the catalyst is supported on an inert carrier preferably alumina, silica, alumina-silica, Alundum, clay or silicon carbide.

8. A process for the upgrading of synthesis gas to obtain selectivity to alkanes and alcohols comprising contacting carbon monoxide and hydrogen in the vapour phase at a reaction temperature of at least 250°C and a reaction pressure of at least 500 psi (3.44 MPa) with a catalyst as claimed in any of claims 1 to 7.

9. A process as claimed in claim 8 characterised in that the ratio of carbon monoxide to hydrogen is 10:1 to 1:10, preferably 3:1 to 1:3.

10. A process as claimed in claim 8 or claim 9 characterised in that the reaction temperature is 275°C to 375°C.

11. A process as claimed in any of claims 8 to 10 characterised in that the reaction pressure is 500 psi to 5000 psi (3.44 to 34.4 MPa).

12. A process as claimed in any of claims 8 to 11 characterised in that it includes as a further step contacting the resulting products of the upgrading of synthesis gas with hydrogen at elevated temperature and pressure in the presence of a hydrogenation catalyst.

13. A process as claimed in claim 12 characterised in that the hydrogenation catalyst is represented by the formula

$$G_eRu_fD_gE_hO_x$$

wherein

G = Zn, Cd and mixtures thereof;
D = Co, Ni, and mixtures thereof;
E = Fe, Cu, Rh, Pd, Os, Ir, Pt and mixtures thereof;

and wherein
e = 0 to 1
f = 0.01 to 1
g = 0.01 to 3,
h = 0 to 1,
x = the number of oxygens determined by the valence requirements of the other elements.

**Patentansprüche**

1. Katalysator der Zusammensetzung

$$A_aRu_bCu_cM'_dN_zO_x$$

worin
A ein Alkalimetall ist,
worin
M' für Rh, Ir, Pd, Pt oder Gemische davon steht und
worin
a 0,02 bis 0,5 ist,
b 0,5 bis 3 ist,
c 0,5 bis 3 ist,
d 0,05 bis 0,5 ist,
z ein Anteil von 0 bis 1 Gew,-% ist und
x die Zahl der benötigten Sauerstoffe ist, um die Valenzanforderungen der anderen Elemente zu erfüllen.

2. Katalysator nach Anspruch 1, dadurch gekennzeichnet, daß A ausgewählt ist aus Natrium, Kalium und Rubidium.
3. Katalysator nach Anspruch 1 oder 2, dadurch gekennzeichnet, daß a 0,02 bis 0,4 ist.
4. Katalysator nach einem der Ansprüche 1 bis 3, dadurch gekennzeichet, daß b und c jeweils 1 sind.
5. Katalysator nach einem der Ansprüche 1 bis 4, dadurch gekennzeichnet, daß d 0,1 bis 0,5 ist.
6. Katalysator nach einem der Ansprüche 1 bis 5, dadurch gekennzeichnet, daß der Katalysator partiell reduziert ist.
7. Katalysator nach einem der Ansprüche 1 bis 6, dadurch gekennzeichnet, daß der Katalysator auf einem inerten Träger, bevorzugt Aluminiumoxid, Siliciumoxid, Aluminiumoxid-Siliciumoxid, Alundum, Ton oder Siliciumcarbid getragen wird.
8. Verfahren zur Verfeinerung von Synthesegas, um Selektivität gegenüber Alkanen und Alkoholen zu erhalten, umfassend Kontakt Herstellen von Kohlenstoffmonoxid und Wasserstoff in der Dampfphase bei einer Reaktionstemperatur von mindestens 250°C und einem Reaktionsdruck von mindestens 500 psi (3,44 MPa) mit einem Katalysator nach einem der Ansprüche 1 bis 7.
9. Verfahren nach Anspruch 8, dadurch gekennzeichnet, daß das Verhältnis von Kohlenstoffmonoxid zu Wasserstoff 10:1 bis 1:10, bevorzugt 3:1 bis 1:3 ist.
10. Verfahren nach Aspruch 8 oder 9, dadurch gekennzeichnet, daß die Reaktionstemperatur 275°C bis 375°C ist.
11. Verfahren nach einem der Ansprüche 8 bis 10, dadurch gekennzeichnet, daß der Reaktionsdruck 500 psi bis 5000 psi (3,44 bis 34,4 MPa) ist.
12. Verfahren nach einem der Ansprüche 8 bis 11, dadurch gekennzeichnet, daß es als einem weiteren Schritt umfaßt Kontakt Herstellen der erhaltenen Produkte der Verfeinerung des Synthesegases mit Wasserstoff bei erhöhter Temperatur und Druck in Gegenwart eines Hydrierungskatalysators.
13. Verfahren nach Anspruch 12, dadurch gekennzeichnet, daß der Hydrierungskatalysator dargestellt ist durch die Formel

$$G_eRu_fD_gE_hO_x$$

worin
G = Zn, Cd und Gemische davon;
D = Co, Ni und Gemische davon;
E = Fe, Cu, Rh, Pd, Os, Ir, Pt und Gemische davon;
und worin
e = 0 bis 1
f = 0,01 bis 3,
g = 0,01 bis 3,
h = 0 bis 1,
x = die Zahl der Sauerstoffe festgelegt durch die Valenzanforderungen der anderen Elemente.

**Revendications**

1. Catalyseur de la composition:

$$A_aRu_bCu_cM'_dN_zO_x$$

où
A est un métal alcalin,
où
M' est Rh, Ir, Pd, Pt ou leurs mélanges et
où
a est 0,02 à 0,5,
b est 0,5 à 3,
c est 0,5 à 3,
d est 0,05 à 0,5,
z est 0 à 1% en poids et
x est le nombre d'oxygène nécessaire pour remplir les conditions de valence des autres éléments.

2. Catalyseur selon la revendication 1, caractérisé en ce que A est choisi parmi le sodium, le potassium et le rubidium.
3. Catalyseur selon la revendication 1 ou la revendication 2, caractérisé en ce que a est 0,02 à 0,4.
4. Catalyseur selon l'une des revendications 1 à 3, caractérisé en ce que b et c sont chacun 1.
5. Catalyseur selon l'une des revendications 1 à 4, caractérisé en ce que d est 0,1 à 0,5.
6. Catalyseur selon l'une des revendications 1 à 5, caractérisé en ce que le catalyseur est partiellement réduit.
7. Catalyseur selon l'une des revendications 1 à 6, caractérisé en ce que le catalyseur est porté sur un support inerte, de préférence de l'alumine, de la silice, de l'alumine-silice, de l'Alundum, de l'argile ou de carbure de silicium.
8. Procédé de valorisation du gaz de synthèse pour obtenir une sélectivité à l'égard des alcanes et des alcools, comportant le fait de mettre en contact de l'oxyde de carbone et de l'hydrogène en phase vapeur à une température de réaction d'au moins 250°C et sous une pression de réaction d'au moins

500 psi (3,44 MPa) avec un catalyseur tel que revendiqué dans l'une quelconque des revendications 1 à 7.

9. Procédé selon la revendication 8, caractérisé en ce que le rapport de l'oxyde de carbone à l'hydrogène est 10:1 à 1:10, de préférence 3:1 à 1:3.

10. Procédé selon la revendication 8 ou la revendication 9, caractérisé en ce que la température de réaction est située entre 275°C et 375°C.

11. Procédé selon l'une des revendications 8 à 10, caractérisé en ce que la pression de réaction est située entre 500 psi et 5000 psi (3,44 et 34,4 MPa).

12. Procédé selon l'une des revendications 8 à 11, caractérisé en ce qu'il comporte comme étape supplémentaire le fait de mettre en contact avec de l'hydrogène les produits résultants de la valorisation du gaz de synthèse, sous température et pression élevées, en présence d'un catalyseur d'hydrogénation.

13. Procédé selon la revendication 12, caractérisé en ce que le catalyseur d'hydrogénation est représenté par fa formule:

$$G_eRu_fD_gE_hO_x$$

où

G = Zn, Cd et leurs mélanges,
D = Co, Ni, et leurs mélanges,
E = Fe, Cu, Rh, Pd,. Os, Ir, Pt et leurs mélanges et où
e = 0 à 1,
f = 0,01 à 3,
g = 01 à 3,
h = 0 à 1,
x = nombre d'oxygènes déterminé par les conditions de valence des autres éléments.